# EUROPÄISCHE PATENTANMELDUNG

(11) **EP 4 775 195 A1**
(43) Veröffentlichungstag der Anmeldung: **15.07.2026**
(21) Anmeldenummer: 25151469.1
(22) Anmeldetag: 13.01.2025
(51) Int. Cl.: A61K 6/30

(54) **STRAHLUNGSHÄRTBARE ZUSAMMENSETZUNG FÜR DEN EINSATZ ALS DENTALES ADHÄSIV**

(71) Anmelder: Kulzer GmbH, 63450 Hanau (DE)
(72) Erfinder: Barther, Dennis, 63843 Niedernberg (DE); Meier, Christoph, 63486 Bruchköbel (DE); Gerlach, Michael, 65719 Hofheim (DE)
(74) Vertreter: Pelster Behrends Patentanwälte PartG mbB

(57) **Zusammenfassung**

Die Erfindung betrifft eine strahlungshärtbare Zusammensetzung für den Einsatz als dentales Adhäsiv, umfassend bezogen auf die Gesamtmasse der strahlungshärtbaren Zusammensetzung: a) eine oder mehrere erste (Meth)acrylamid-Verbindungen in einem kombinierten Massenanteil von 1 % oder mehr, wobei die ersten (Meth)acrylamid-Verbindungen bei einem Druck von 100 kPa einen Schmelzpunkt von 30 °C oder mehr aufweisen, b) eine oder mehrere zweite (Meth)acrylamid-Verbindungen in einem kombinierten Massenanteil von 0,1 % oder mehr, wobei die zweiten (Meth)acrylamid-Verbindungen bei einem Druck von 100 kPa einen Schmelzpunkt von 20 °C oder weniger aufweisen, und c) einen oder mehrere Photoinitiatoren in einem kombinierten Massenanteil im Bereich von 0,001 bis 10 %.

## Beschreibung

Die Erfindung betrifft eine strahlungshärtbare Zusammensetzung für den Einsatz als dentales Adhäsiv und eine damit herstellbare dentale Verklebung. Offenbart wird zudem die Verwendung eines (Meth)acrylamid-Gemischs bei der Herstellung von strahlungshärtbaren dentalen Adhäsiven zur Verringerung der Abnahme der Scherhaftung des dentalen Adhäsivs an Dentin und/oder Enamel mit der Zeit.

Der Bereich der restaurativen Zahnmedizin umfasst viele Methoden zur Behandlung und insbesondere Wiederherstellung von erkrankten oder beschädigten Zähnen. Eine wichtige Behandlungsmethode ist hierbei beispielsweise die Entfernung von Zahnsubstanz, nach der das so entstandene Loch mit einer Füllung versehen wird. Neben Füllungen aus Metalllegierungen und keramischen Materialien werden dafür heutzutage insbesondere hochspezialisierte Kunststoffprodukte eingesetzt. Solche Füllungskunststoffe werden dabei regelmäßig aus strahlungshärtbaren Zusammensetzungen durch Polymerisation erhalten, sodass eine mechanisch belastbare Füllung resultiert.

Neben den mechanischen Eigenschaften des Füllungsmaterials selbst ist es für eine erfolgreiche Therapie essentiell, dass die in einem Loch platzierte Füllung einen festen Sitz aufweist und auch unter den im Alltag zu erwartenden Belastungen eine hohe Langlebigkeit aufweist, sodass sich die Füllung beispielsweise nicht etwa mit der Zeit aus dem Loch löst.

Um diese gewünschte Haftung der Füllung am Zahnmaterial zu gewährleisten, werden sogenannte dentale Adhäsive eingesetzt. Diese dentalen Adhäsive sind in den meisten Fällen ebenfalls aushärtbare Kunststoffe, die hinsichtlich ihrer physikalisch-chemischen Eigenschaften jedoch insbesondere daraufhin optimiert werden, eine gute Adhäsion sowohl zu dem unterliegenden Zahnmaterial als auch zu der darauf applizierten Füllung auszubilden.

Dentale Adhäsive im Allgemeinen und auch solche, welche auf (Meth)acrylamid-Verbindungen basieren, sind dem Fachmann grundsätzlich bekannt, wobei hinsichtlich des Einsatzes verschiedene Konzepte zum Einsatz kommen, beispielsweise das sogenannte "Self-Etch" oder das "Etch-and-Rinse". Beispiele für dentale Adhäsive sind im Stand der Technik beispielsweise in der WO 2017/017155 A1, der EP 3156033 A1, der US 20240052079 A1 oder der US 11712404 B2 offenbart.

Nach Einschätzung der Erfinder sind mit vielen der aus dem Stand der Technik bekannten dentalen Adhäsive bereits heute in vielen Fällen gute Ergebnisse zu erzielen, insbesondere mit dentalen Adhäsiven, die auf den Einsatz von (Meth)acrylamiden setzen. Allerdings werden die aus dem Stand der Technik bekannten dentalen Adhäsive hinsichtlich einiger Aspekte zuweilen als verbesserungsbedürftig empfunden. Insbesondere bestehen für dentale Adhäsive regelmäßig hohe Anforderungen an die Anwendungseigenschaften, wobei insbesondere zahlreiche Zielkonflikte bestehen, bei denen bestimmte Eigenschaften nicht, beziehungsweise nur schwer, gleichzeitig zu optimieren sind.

Eine wichtige Eigenschaft dentaler Adhäsive, für die eine Optimierung wünschenswert ist, ist die Scherfestigkeit der entsprechenden dentalen Adhäsive, welche unmittelbar auf der Zahnsubstanz bestimmt werden kann, und deren langfristige Entwicklung. Insoweit wurde gefunden, dass zahlreiche (Meth)acrylamide, welche hinsichtlich ihrer sonstigen Eigenschaften, beispielsweise der Verarbeitungseigenschaften und/oder des Polymerisationsverhaltens, für den Einsatz in dentalen Adhäsiven grundsätzlich wünschenswert wären, mit Blick auf die erzielbare Scherfestigkeit in vielen Fällen nicht den hohen Anforderungen genügen. Dadurch ist die dem Fachmann für die Formulierung von hochleistungsfähigen (Meth)acrylamid-basierten dentalen Adhäsiven zur Verfügung stehende Palette an Ausgangsmaterialien und damit die Flexibilität bei der Optimierung der physikalisch-chemischen sowie mechanischen Eigenschaften der dentalen Adhäsive begrenzt.

Insbesondere bei den festen (Meth)acrylamid-Verbindungen, welche grundsätzlich eine sehr interessante Klasse von Verbindungen für den Einsatz in dentalen Adhäsiven darstellen, wird in vielen Fällen gefunden, dass die damit erzielbare Scherfestigkeit für viele Anwendungen bereits initial unzureichend ist. Bessere initiale Scherfestigkeiten können in einigen Fällen erhalten werden, wenn statt der festen (Meth)acrylamide flüssige (Meth)acrylamide eingesetzt werden. Allerdings zeigt sich auch hierbei, dass nicht mit sämtlichen flüssigen (Meth)acrylamiden die an sich gewünschten Ergebnisse erzielt werden, sodass auch in diesem Falle die Formulierung von hochleistungsfähigen dentalen Adhäsiven für den Fachmann mit einem großen Versuchsaufwand verbunden sein kann, um geeignete flüssige (Meth)acrylamide zu identifizieren.

Flüssige (Meth)acrylamide haben darüber hinaus in vielen Fällen auch den Nachteil, dass diese hinsichtlich der Beständigkeit der Scherfestigkeit der erzeugten Verklebung unter thermischer Belastung zuweilen als unzureichend empfunden werden, insbesondere auch im Vergleich mit vielen festen (Meth)acrylamiden, die bei einer zumeist niedrigeren initialen Scherfestigkeit häufig zumindest eine bessere Dauerhaftigkeit zeigen und darüber hinaus auch mit Blick auf die die generellen Eigenschaften der mittels der dentalen Adhäsive zu erhaltenden dentalen Verklebung günstigere Ergebnisse liefern als die flüssigen (Meth)acrylamide.

Die primäre Aufgabe der vorliegenden Erfindung war es, die Nachteile des Standes der Technik auszuräumen oder zumindest abzuschwächen.

Insbesondere war es die Aufgabe der vorliegenden Erfindung, leistungsfähige, strahlungshärtbare Zusammensetzungen für den Einsatz in dentalen Adhäsiven anzugeben, mit denen sich in zuverlässiger Weise hochleistungsfähige dentale Verklebungen herstellen lassen.

Insoweit war es eine Aufgabe der vorliegenden Erfindung, dass die anzugebenden dentalen Adhäsive an den relevanten Zahnmaterialien insbesondere eine ausgezeichnete initiale Scherfestigkeit aufweisen sollen.

Darüber hinaus war es eine weitere Aufgabe der vorliegenden Erfindung, dass die anzugebenden dentalen Adhäsive an entsprechenden Zahnmaterialien zudem auch nach längerer Zeit, insbesondere auch unter thermischer Belastung, eine möglichst gleichbleibende Scherfestigkeit aufweisen sollten, sodass mit den anzugebenden dentalen Adhäsiven langlebige und thermisch belastbare dentale Verklebungen herstellbar sein sollten.

Zudem war es eine Aufgabe der vorliegenden Erfindung, dass die anzugebenden dentalen Adhäsive vorteilhafte Verarbeitungseigenschaften und eine günstige Aushärtekinetik aufweisen sollten.

Insbesondere war es eine weitere Aufgabe der vorliegenden Erfindung, dass die anzugebende Lösung es ermöglichen sollte, eine breitere Palette an chemischen Verbindungen für den Einsatz in leistungsfähigen dentalen Adhäsiven zugänglich zu machen, um hiermit die Flexibilität des Fachmannes bei der Formulierung leistungsfähiger dentaler Adhäsive zu erhöhen und die Rohstoffbasis zu verbreitern.

Zudem war es eine Aufgabe der vorliegenden Erfindung, dass die anzugebende Lösung möglichst weitgehend ohne den Einsatz von potenziell umwelt- und/oder gesundheitsschädlichen Substanzen auskommen sollte, wobei es eine wünschenswerte Maßgabe war, dass die anzugebenden dentalen Adhäsive möglichst weitgehend mit solchen Verfahren und Vorrichtungen herstellbar sein sollten, die bereits heute bei der Formulierung dentaler Adhäsive zum Einsatz kommen.

Es war vor diesem Hintergrund eine weitere Aufgabe der vorliegenden Erfindung, dass das anzugebende dentale Adhäsiv möglichst kosteneffizient herstellbar sein sollte.

Zudem war es eine Aufgabe der vorliegenden Erfindung, eine mit dem anzugebenden dentalen Adhäsiv herstellbare dentale Verklebung bereitzustellen.

Es war eine sekundäre Aufgabe der vorliegenden Erfindung, zudem eine vorteilhafte Verwendung eines spezifischen (Meth)acrylamid-Gemischs bei der Herstellung von strahlungshärtbaren dentalen Adhäsiven zur Verringerung der Abnahme der Scherhaftung des dentalen Adhäsives an Dentin und/oder Enamel mit der Zeit bereitzustellen.

Die Erfinder der vorliegenden Erfindung haben nunmehr gefunden, dass sich die vorstehend beschriebenen Aufgaben überraschenderweise lösen lassen, wenn eine strahlungshärtbare Zusammensetzung für den Einsatz als dentales Adhäsiv bereitgestellt wird, welche neben Photoinitiatoren auch zwei unterschiedliche Arten von (Meth)acrylamid-Verbindungen umfasst, von denen die erste der (Meth)acrylamid-Verbindungen bei Raumtemperatur ein Feststoff ist, wohingegen die andere (Meth)acrylamid-Verbindung bei Raumtemperatur flüssig bzw. viskos ist.

Überraschenderweise lässt sich durch den kombinierten Einsatz von bei Raumtemperatur festen und flüssigen (Meth)acrylamid-Verbindungen in vorteilhafter Weise eine strahlungshärtbare Zusammensetzung erhalten, welche nicht nur eine ausgezeichnete initiale Scherfestigkeit zeigt, welche im Vergleich mit der gleichen Menge an Einzelsubstanzen synergistisch verbessert ist, sondern es wird überraschenderweise auch eine verbesserte Beständigkeit der Scherfestigkeit unter thermischer Beanspruchung erreicht.

Völlig unerwartet ist hierbei, dass sich durch die erfindungsgemäß vorgesehene Kombination von festen und flüssigen (Meth)acrylamid-Verbindungen, insbesondere auch mit solchen (Meth)acrylamid-Verbindungen, welche bei der isolierten Verwendung unzureichende Scherfestigkeiten zeigen, plötzlich ausgezeichnete Ergebnisse erzielen lassen, sodass diese (Meth)acrylamid-Verbindungen durch die erfindungsgemäße Kombination überraschenderweise zugänglich für die Formulierung von hochleistungsfähigen dentalen Adhäsiven werden, wodurch die Flexibilität des mit der Formulierung von dentalen Adhäsiven beauftragten Fachmanns bei der Entwicklung neuer dentaler Adhäsive signifikant gesteigert wird.

Ohne an diese Theorie gebunden sein zu wollen, gehen die Erfinder ausgehend von den durchgeführten Experimenten davon aus, dass viele der beim Einsatz von aus dem Stand der Technik bekannten festen (Meth)acrylamid-Verbindungen auftretenden Probleme, insbesondere hinsichtlich der geringen initialen Scherfestigkeiten, darauf zurückzuführen sind, dass die zuvor gelösten aber inhärent festen (Meth)acrylamid-Verbindungen nach dem Entfernen des Lösungsmittels der dentalen Adhäsive eine Rekristallisation zeigen, was auf die Fähigkeit zur Ausbildung von Wasserstoffbrücken-Bindungen zurückgeführt wird, wobei die Erfinder davon ausgehen, dass diese Rekristallisation maßgeblich für die verschlechterten Scherfestigkeiten ist, da die Fähigkeit zur Erzeugung einer homogenen Adhäsivschicht nach Entfernen des Lösungsmittels essentiell für eine stabile Haftung ist. Wiederum ohne an diese Theorie gebunden sein zu wollen vermuten die Erfinder, dass die angenommene Abhängigkeit der Scherfestigkeiten von den Kristallisationseigenschaften auch mit dafür verantwortlich ist, dass viele der festen (Meth)acrylamid-Verbindungen ein schwer vorhersehbares Verhalten bei den erreichbaren Scherfestigkeiten zeigen. Ausgehend von dieser Annahme ließe sich auch erklären, warum viele flüssige (Meth)acrylamid-Verbindungen bessere initiale Scherfestigkeiten zeigen, da bei diesen die Rekristallisation entfällt.

Ohne an diese Theorie gebunden sein zu wollen, gehen die Erfinder davon aus, dass in erfindungsgemäß zusammengesetzten strahlungshärtbaren Zusammensetzungen die flüssigen (Meth)acrylamid-Verbindungen in vorteilhafter Weise quasi als Lösungsmittel für die festen (Meth)acrylamid-Verbindungen fungieren beziehungsweise in der Wechselwirkung zumindest einer Rekristallisation der festen (Meth)acrylamid-Verbindungen entgegenwirken, so dass die mit der Rekristallisation einhergehenden Nachteile gemindert oder sogar vollständig verhindert werden können.

Die vorstehend genannten Aufgaben werden somit durch den Gegenstand der Erfindung gelöst, wie er in den Ansprüchen definiert ist. Bevorzugte erfindungsgemäße Ausgestaltungen ergeben sich aus den Unteransprüchen und den nachfolgenden Ausführungen.

Solche Ausführungsformen, die nachfolgend als bevorzugt bezeichnet sind, werden in besonders bevorzugten Ausführungsformen mit Merkmalen anderer als bevorzugt bezeichneter Ausführungsformen kombiniert. Ganz besonders bevorzugt sind somit Kombinationen von zwei oder mehr der nachfolgend als besonders bevorzugt bezeichneten Ausführungsformen. Ebenfalls bevorzugt sind Ausführungsformen, in denen ein in irgendeinem Ausmaß als bevorzugt bezeichnetes Merkmal einer Ausführungsform mit einem oder mehreren weiteren Merkmalen anderer Ausführungsformen kombiniert wird, die in irgendeinem Ausmaß als bevorzugt bezeichnet werden. Merkmale bevorzugter dentaler Verklebungen und Verwendungen ergeben sich aus den Merkmalen bevorzugter strahlungshärtbarer Zusammensetzungen.

Insoweit nachfolgend für ein Element, beispielsweise für die ersten (Meth)acrylamid-Verbindungen oder die Photoinitiatoren, sowohl spezifische Mengen bzw. Anteile dieses Elementes als auch bevorzugte Ausgestaltungen des Elementes offenbart werden, sind insbesondere auch die spezifischen Mengen bzw. Anteile der bevorzugt ausgestalteten Elemente offenbart. Zudem ist offenbart, dass bei den entsprechenden spezifischen Gesamtmengen bzw. Gesamtanteilen der Elemente zumindest ein Teil der Elemente bevorzugt ausgestaltet sein kann und insbesondere auch, dass bevorzugt ausgestaltete Elemente innerhalb der spezifischen Gesamtmengen oder Gesamtanteile wiederum in den spezifischen Mengen bzw. Anteilen vorliegen können.

Insbesondere bevorzugte Ausführungsformen der Erfindung sind in den Ausführungsbeispielen offenbart. Besonders bevorzugte Ausführungsformen der Erfindung weisen vor diesem Hintergrund zwei oder mehr, bevorzugt drei oder mehr, ganz besonders bevorzugt vier oder mehr, der nachfolgend offenbarten bevorzugten Merkmale der Erfindung auf, welche auch in den Ausführungsbeispielen realisiert sind.

Die Erfindung betrifft insbesondere eine strahlungshärtbare Zusammensetzung für den Einsatz als dentales Adhäsiv, umfassend bezogen auf die Gesamtmasse der strahlungshärtbaren Zusammensetzung:
a) eine oder mehrere erste (Meth)acrylamid-Verbindungen in einem kombinierten Massenanteil von 1 % oder mehr,
   wobei die ersten (Meth)acrylamid-Verbindungen bei einem Druck von 100 kPa einen Schmelzpunkt von 30 °C oder mehr aufweisen,
b) eine oder mehrere zweite (Meth)acrylamid-Verbindungen in einem kombinierten Massenanteil von 0,1 % oder mehr,
   wobei die zweiten (Meth)acrylamid-Verbindungen bei einem Druck von 100 kPa einen Schmelzpunkt von 20 °C oder weniger aufweisen, und
c) einen oder mehrere Photoinitiatoren in einem kombinierten Massenanteil im Bereich von 0,001 bis 10 %.

Die Erfindung betrifft eine strahlungshärtbare Zusammensetzung, welche für den Einsatz als dentales Adhäsiv geeignet und bestimmt ist und mit dem eine dentale Verklebung erzeugt werden kann, beispielsweise um eine Zahnfüllung stoffschlüssig an Zahnsubstanz zu befestigen. Dentale Adhäsive im Allgemeinen sowie Verfahren zu deren Herstellung und die Grundkonzepte ihrer Verwendung sind dem Fachmann im dentalen Bereich gut vertraut.

Im Rahmen der vorliegenden Erfindung werden die definierten Bestandteile der strahlungshärtenden Zusammensetzung in Übereinstimmung mit dem fachmännischen Verständnis jeweils als "ein oder mehrere" eingesetzt. Die Bezeichnung "ein oder mehrere" bezieht sich dabei in branchenüblicher Weise auf die chemische Natur der entsprechenden Verbindungen und nicht auf deren Stoffmenge.

Insoweit im Rahmen der Erfindung Massenanteile angegeben werden, werden diese in branchenüblicher Weise jeweils als kombinierte Massenanteile der einen oder der mehreren Komponenten angegeben, wodurch ausgedrückt wird, dass der Massenanteil der entsprechend ausgebildeten Komponenten zusammengenommen die entsprechenden Kriterien erfüllt.

Soweit nicht im Einzelfall etwas anderes definiert wird, beziehen sich die im Rahmen der vorliegenden Erfindung definierten Massenanteile für die Komponenten der strahlungshärtbaren Zusammensetzung jeweils auf die Gesamtmasse der strahlungshärtbaren Zusammensetzung. Der Fachmann versteht dabei, dass die Massenanteile mit der Maßgabe definiert werden, dass die Gesamtmassenanteile der strahlungshärtbaren Zusammensetzung sich zu 100 % addieren, so dass definierte Obergrenzen eines Bestandteils im Bedarfsfall anzupassen sind, so dass sie zusammen mit den Untergrenzen der weiteren verpflichtenden Bestandteile 100 % ergeben.

Die Erfindung basiert insbesondere auf dem Einsatz von spezifischen (Meth)acrylamid-Verbindungen. In Übereinstimmung mit dem Verständnis des Fachmanns bezeichnet der Ausdruck (Meth)acrylamid dabei sowohl Acrylamide als auch Methacrylamide. Im Bereich der Dentalchemie sind darüber hinaus insbesondere auch (Meth)acrylate als Monomere von herausragender Bedeutung, für welche die Auslegung der Nomenklatur entsprechend zutrifft.

Die erfindungsgemäßen strahlungshärtbaren Zusammensetzungen setzen auf den kombinierten Einsatz von zwei unterschiedlichen Arten von (Meth)acrylamid-Verbindung, welche im Rahmen der vorliegenden Erfindung als erste (Meth)acrylamid-Verbindung und zweite (Meth)acrylamid-Verbindungen bezeichnet werden, und welche sich hinsichtlich ihres Schmelzpunktes und damit hinsichtlich ihres Aggregatzustands bei Raumtemperatur unterscheiden.

In der vorstehenden Definition werden jeweils Mindestgehalte für den Massenanteil der jeweiligen (Meth)acrylamid-Verbindungen definiert, oberhalb derer sich die Vorteile der Erfindung deutlich zeigen. Durch die vorstehend definierten Untergrenzen wird der Gegenstand der Erfindung zudem klar von solchen Zusammensetzungen abgegrenzt, welche (Meth)acrylamid-Verbindungen lediglich in geringen Mengen enthalten, beispielsweise als Neben- und/oder Zersetzungsprodukte. Den Erfindern ist es jedoch darüber hinaus gelungen, für den Gehalt an ersten (Meth)acrylamid-Verbindungen und zweiten (Meth)acrylamid-Verbindungen jeweils besonders bevorzugte Werte bzw. Bereiche zu identifizieren.

Bevorzugt ist hinsichtlich der ersten (Meth)acrylamid-Verbindungen nämlich eine erfindungsgemäße strahlungshärtbare Zusammensetzung, wobei der kombinierte Massenanteil der einen oder der mehreren ersten (Meth)acrylamid-Verbindungen 2 % oder mehr, bevorzugt 4 % oder mehr, besonders bevorzugt 6 % oder mehr, ganz besonders bevorzugt 8 % oder mehr, insbesondere bevorzugt 10 % oder mehr, beträgt, bezogen auf die Gesamtmasse der strahlungshärtbaren Zusammensetzung, und/oder wobei der kombinierte Massenanteil der einen oder der mehreren ersten (Meth)acrylamid-Verbindungen 30 % oder weniger, bevorzugt 25 % oder weniger, besonders bevorzugt 20 % oder weniger, ganz besonders bevorzugt 15 % oder weniger, beträgt, bezogen auf die Gesamtmasse der strahlungshärtbaren Zusammensetzung. Besonders bevorzugt ist entsprechend eine erfindungsgemäße strahlungshärtbare Zusammensetzung, wobei der kombinierte Massenanteil der einen oder der mehreren ersten (Meth)acrylamid-Verbindungen im Bereich von 2 bis 30 %, bevorzugt im Bereich von 4 bis 25 %, besonders bevorzugt im Bereich von 6 bis 25 %, liegt, ganz besonders bevorzugt im Bereich von 10 bis 15 %, bezogen auf die Gesamtmasse der strahlungshärtbaren Zusammensetzung.

Bevorzugt ist bezüglich der zweiten (Meth)acrylamid-Verbindungen eine erfindungsgemäße strahlungshärtbare Zusammensetzung, wobei der kombinierte Massenanteil der einen oder der mehreren zweiten (Meth)acrylamid-Verbindungen 0,2 % oder mehr, bevorzugt 0,4 % oder mehr, besonders bevorzugt 0,6 % oder mehr, ganz besonders bevorzugt 0,8 % oder mehr, insbesondere bevorzugt 1 % oder mehr, beträgt, bezogen auf die Gesamtmasse der strahlungshärtbaren Zusammensetzung, und/oder wobei der kombinierte Massenanteil der einen oder der mehreren zweiten (Meth)acrylamid-Verbindungen 10 % oder weniger, bevorzugt 8 % oder weniger, besonders bevorzugt 6 % oder weniger, ganz besonders bevorzugt 4 % oder weniger, beträgt, bezogen auf die Gesamtmasse der strahlungshärtbaren Zusammensetzung. Besonders bevorzugt ist entsprechend eine erfindungsgemäße strahlungshärtbare Zusammensetzung, wobei der kombinierte Massenanteil der einen oder der mehreren zweiten (Meth)acrylamid-Verbindungen im Bereich von 0,2 bis 10 %, bevorzugt im Bereich von 0,4 bis 8 %, besonders bevorzugt im Bereich von 0,6 bis 6 %, liegt, ganz besonders bevorzugt im Bereich von 0,8 bis 4 %, bezogen auf die Gesamtmasse der strahlungshärtbaren Zusammensetzung.

Die Erfinder der vorliegenden Erfindung haben es als besonders vorteilhaft identifiziert, die spezifischen (Meth)acrylamid-Verbindungen in den erfindungsgemäßen strahlungshärtbaren Zusammensetzungen in unterschiedlichen Verhältnissen einzusetzen, wobei besonders leistungsfähige strahlungshärtbare Zusammensetzungen erhalten werden können, wenn die festen (Meth)acrylamid-Verbindungen, d. h. die ersten (Meth)acrylamid-Verbindungen, im Überschuss eingesetzt werden, wobei es insbesondere bevorzugt ist, relativ große Überschüsse vorzusehen. In den Experimenten der Erfinder hat sich dabei eindrucksvoll gezeigt, dass sich bereits bei der Zugabe kleiner Mengen der flüssigen (Meth)acrylamid-Verbindungen ausgezeichnete Scherfestigkeiten erhalten lassen, wobei im Überschuss der festen (Meth)acrylamid-Verbindungen besonders große synergistische Effekte beobachtet werden konnten. Da darüber hinaus auch unter anwendungstechnischen Gesichtspunkten sowie unter dem Aspekt der Haltbarkeit der dentalen Verklebung, insbesondere auch unter thermischer Wechselbelastung, der Einsatz von festen (Meth)acrylamid-Verbindungen günstig ist, erachten die Erfinder diese Ausführungsform als besonders bevorzugt. Bevorzugt ist entsprechend eine erfindungsgemäße strahlungshärtbare Zusammensetzung, wobei der Quotient aus dem kombinierten Massenanteil an ersten (Meth)acrylamid-Verbindungen geteilt durch den kombinierten Massenanteil an zweiten (Meth)acrylamid-Verbindungen 1 oder mehr, bevorzugt 2 oder mehr, besonders bevorzugt 4 oder mehr, beträgt.

In den Experimenten der Erfinder hat sich bei der Einstellung des Verhältnisses jedoch auch gezeigt, dass es unter anderen Gesichtspunkten zweckmäßig sein kann, den Anteil der flüssigen (Meth)acrylamid-Verbindungen nicht zu klein werden zu lassen, sodass diese ihren vorteilhaften Einfluss ausüben können. Bevorzugt ist demgemäß eine erfindungsgemäße strahlungshärtbare Zusammensetzung, wobei der Quotient aus dem kombinierten Massenanteil an ersten (Meth)acrylamid-Verbindungen geteilt durch den kombinierten Massenanteil an zweiten (Meth)acrylamid-Verbindungen 20 oder weniger, bevorzugt 15 oder weniger, besonders bevorzugt 10 oder weniger, beträgt.

Besonders bevorzugt ist im Lichte der vorstehenden Ausführungen eine erfindungsgemäße strahlungshärtbare Zusammensetzung, wobei der Quotient aus dem kombinierten Massenanteil an ersten (Meth)acrylamid-Verbindungen geteilt durch den kombinierten Massenanteil an zweiten (Meth)acrylamid-Verbindungen im Bereich von 1 bis 20, bevorzugt im Bereich von 2 bis 15, besonders bevorzugt im Bereich von 4 bis 10, liegt.

Wie vorstehend erläutert, unterscheiden sich die ersten (Meth)acrylamid-Verbindungen und die zweiten (Meth)acrylamid-Verbindungen hinsichtlich der Schmelzpunkte und dadurch mittelbar darin, ob sie bei Raumtemperatur, d. h. im Rahmen der vorliegenden Erfindung bei 25 °C, Feststoffe oder Flüssigkeiten sind. In Übereinstimmung mit dem fachmännischen Verständnis wird dies auf die Natur der reinen Stoffe bezogen und nicht auf den Zustand der strahlungshärtbaren Zusammensetzung. Ein bei Raumtemperatur festes (Meth)acrylamid kann in der erfindungsgemäßen strahlungshärtbaren Zusammensetzung somit als Teil der Flüssigkeit gelöst vorliegen.

Zum Zwecke einer klaren Definition werden die ersten und zweiten (Meth)acrylamid-Verbindungen über den Schmelzpunkt, d. h. den Schmelzpunkt der Reinsubstanzen unterschieden, wobei die vorstehende Definition durch einen gewissen Abstand der Schmelzpunkte um die Temperatur 25 °C herum, eine klare Unterscheidung erlaubt. In Übereinstimmung mit dem fachmännischen Verständnis ist der Schmelzpunkt die Temperatur, bei der eine chemische Verbindung vom festen in den flüssigen Aggregatzustand übergeht. Bei dem Schmelzpunkt handelt es sich dabei um eine der Standardgrößen, welche vom Fachmann zur Charakterisierung von Stoffen verwendet wird und die dem Fachmann aus den frühesten Tagen seines Studiums bekannt ist.

Auch wenn der Schmelzpunkt üblicherweise nur im geringen Maße vom Druck abhängig ist, wird vorstehend der Vollständigkeit halber definiert, dass der Schmelzpunkt bei 100 kPa heranzuziehen ist.

Den im Wesentlichen nicht von den Messbedingungen abhängigen Schmelzpunkt kann der Fachmann in üblicher Weise an der reinen Substanz ermitteln, in einfachen Ausgestaltungen beispielsweise unter Einsatz eines üblichen Schmelzpunktbestimmungsröhrchens. Hierfür wird die zu untersuchende Substanz in ein kapillares Schmelzpunktbestimmungsröhrchen eingefüllt, welches anschließend in einer geeigneten Heizvorrichtung kontrolliert und langsam einer Temperaturerhöhung unterzogen wird, wobei die entsprechende Vorrichtung üblicherweise ein Sichtfenster aufweist, durch das hindurch der Fachmann visuell bewerten kann, an welchem Zeitpunkt es zu einem Aufschmelzen der untersuchten Substanz kommt. Die von dem Gerät zu diesem Zeitpunkt des Aufschmelzens angegebene Temperatur kann als Schmelzpunkt notiert werden. Bevorzugt wird der Schmelzpunkt jedoch mittels dynamischer Differenzkalorimetrie (Heizrate 20 K/min) bestimmt. Sofern bei der Temperaturerhöhung bei einer Temperatur Tz eine Zersetzung beobachtet wird, bevor es zu einem Aufschmelzen der untersuchten Substanz kommt, bedeutet dies im Rahmen der vorliegenden Erfindung in Übereinstimmung mit dem fachmännischen Verständnis, dass der Schmelzpunkt der untersuchten Substanz oberhalb von Tz liegt.

Die Erfinder schlagen vor, dass zu einer klaren Unterscheidung der Substanzen und für einen ausgeprägten Effekt der Wechselwirkung zwischen festen und flüssigen (Meth)acrylamid-Verbindungen eine größere Differenz in den Schmelzpunkten eingestellt werden kann. Bevorzugt ist insoweit eine erfindungsgemäße strahlungshärtbare Zusammensetzung, wobei die ersten (Meth)acrylamid-Verbindungen bei einem Druck von 100 kPa einen Schmelzpunkt von 40 °C oder mehr, bevorzugt 50 °C oder mehr, besonders bevorzugt 60 °C oder mehr, aufweisen. Bevorzugt ist zusätzlich oder alternativ eine erfindungsgemäße strahlungshärtbare Zusammensetzung, wobei die zweiten (Meth)acrylamid-Verbindungen bei einem Druck von 100 kPa einen Schmelzpunkt von 10 °C oder weniger, bevorzugt 0 °C oder weniger, besonders bevorzugt -10 °C oder weniger, aufweisen.

Auch wenn die vorstehende Definition über die Schmelzpunkte eine besonders präzise Bestimmung erlaubt, ist es mit Blick auf die spätere Praxis für den Fachmann erfreulicherweise sehr leicht qualitativ festzustellen, ob eine vorgegebene (Meth)acrylamid-Verbindung fest oder flüssig ist, da sich für die weit überwiegende Zahl der relevanten (Meth)acrylamid-Verbindungen diese Frage zuverlässig qualitativ durch eine fachmännische Inaugenscheinnahme bewerten lässt. In der Praxis handelt es sich in der weit überwiegenden Zahl der Fälle um eine erfindungsgemäße strahlungshärtbare Zusammensetzung, wobei die ersten (Meth)acrylamid-Verbindungen bei einem Druck von 100 kPa bei 25 °C Feststoffe sind. Entsprechend sind erfindungsgemäß strahlungshärtbare Zusammensetzungen besonders relevant, wobei die zweiten (Meth)acrylamid-Verbindungen bei einem Druck von 100 kPa bei 25 °C flüssig sind, und/oder wobei die zweiten (Meth)acrylamid-Verbindungen bei einem Druck von 100 kPa bei 25 °C eine dynamische Viskosität gemessen gemäß DIN 53019-1 aus 2008 von 50 Pa s oder weniger, bevorzugt 10 Pa s oder weniger, besonders bevorzugt 1 Pa s oder weniger, aufweisen.

Offenbart wird mit Blick auf eine einfache Definition im Rahmen der vorliegenden Erfindung zudem auch eine strahlungshärtbare Mischung für den Einsatz als dentales Adhäsiv, umfassend bezogen auf die Gesamtmasse der strahlungshärtbaren Mischung:
a) eine oder mehrere erste (Meth)acrylamid-Verbindungen in einem kombinierten Massenanteil von 1 % oder mehr,
   wobei die ersten (Meth)acrylamid-Verbindungen bei einem Druck von 100 kPa bei 25 °C Feststoffe sind,
b) eine oder mehrere zweite (Meth)acrylamid-Verbindungen in einem kombinierten Massenanteil von 0,1 % oder mehr,
   wobei die zweiten (Meth)acrylamid-Verbindungen bei einem Druck von 100 kPa bei 25 °C flüssig sind, und/oder bei einem Druck von 100 kPa bei 25 °C eine dynamische Viskosität gemessen gemäß DIN 53019-1 aus 2008 von 50 Pa s oder weniger, bevorzugt 10 Pa s oder weniger, besonders bevorzugt 1 Pa s oder weniger, aufweisen, und
c) einen oder mehrere Photoinitiatoren in einem kombinierten Massenanteil im Bereich von 0,001 bis 10 %.

Die vorstehend offenbarte strahlungshärtbare Mischung ist vorteilhaft, da sie eine sehr einfache, qualitative und doch für den Fachmann klare Definition für die der Erfindung zugrunde liegende Idee umfasst, sodass sie auch aus sich heraus vorteilhaft ist. Bevorzugte Merkmale der offenbarten strahlungshärtbaren Mischung ergeben sich analog zu den bevorzugten Merkmalen erfindungsgemäßer strahlungshärtbarer Zusammensetzungen.

Nach Einschätzung der Erfinder ergeben sich besonders große Vorteile der erfindungsgemäßen Kombination an festen und flüssigen (Meth)acrylamid-Verbindungen beim Einsatz von Methacrylamid-Verbindungen. Nach Erkenntnis der Erfinder gilt dies insbesondere, wenn für die festen (Meth)acrylamid-Verbindungen die Methacrylamide eingesetzt werden. Ohne an diese Theorie gebunden sein zu wollen, gehen die Erfinder davon aus, dass dies insbesondere der stärkeren Kristallisationsneigung von Methacrylamiden gegenüber Acrylamiden zuzuschreiben ist, sodass feste Methacrylamide stärker von der erfindungsgemäß vorgesehenen Kombination mit den zweiten (Meth)acrylamid-Verbindungen profitieren. Bevorzugt ist folglich eine erfindungsgemäße strahlungshärtbare Zusammensetzung, wobei die ersten (Meth)acrylamid-Verbindungen ausgewählt sind aus der Gruppe bestehend aus Methacrylamiden. Bevorzugt ist zusätzlich oder alternativ auch eine erfindungsgemäße strahlungshärtbare Zusammensetzung, wobei die zweiten (Meth)acrylamid-Verbindungen ausgewählt sind aus der Gruppe bestehend aus Methacrylamiden.

Es kann als Vorteil der vorliegenden Erfindung gesehen werden, dass sich insbesondere auch bei monofunktionellen (Meth)acrylamid-Verbindungen vorteilhafte Eigenschaften gezeigt haben, insbesondere wenn diese als flüssige (Meth)acrylamid-Verbindungen eingesetzt werden. Mit Blick auf die Verarbeitungseigenschaften, die Reaktionskinetik sowie die mechanischen Eigenschaften der damit herstellbaren dentalen Verklebung erachten die Erfinder jedoch prinzipiell den Einsatz von polyfunktionellen (Meth)acrylamid-Verbindungen als bevorzugt. Bevorzugt ist entsprechend eine erfindungsgemäße strahlungshärtbare Zusammensetzung, wobei die ersten (Meth)acrylamid-Verbindungen ausgewählt sind aus der Gruppe bestehend aus polyfunktionellen (Meth)acrylamid-Verbindungen, bevorzugt difunktionellen (Meth)acrylamid-Verbindungen und trifunktionellen (Meth)acrylamid-Verbindungen, besonders bevorzugt difunktionellen (Meth)acrylamid-Verbindungen. Bevorzugt ist zusätzlich oder alternativ auch eine erfindungsgemäße strahlungshärtbare Zusammensetzung, wobei die zweiten (Meth)acrylamid-Verbindungen ausgewählt sind aus der Gruppe bestehend aus polyfunktionellen (Meth)acrylamid-Verbindungen, bevorzugt difunktionellen (Meth)acrylamid-Verbindungen und trifunktionellen (Meth)acrylamid-Verbindungen, besonders bevorzugt difunktionellen (Meth)acrylamid-Verbindungen.

Aufbauend auf den Erkenntnissen der Erfinder ist es diesen gelungen, für die ersten (Meth)acrylamid-Verbindungen sowie die zweiten (Meth)acrylamid-Verbindungen jeweils bevorzugte Strukturen anzugeben, mit denen in erfindungsgemäßen strahlungshärtbaren Zusammensetzungen besonders vorteilhafte Ergebnisse erzielt werden konnten.

Bevorzugt ist bezüglich der ersten (Meth)acrylamid-Verbindungen zunächst eine erfindungsgemäße strahlungshärtbare Zusammensetzung, wobei die ersten (Meth)acrylamid-Verbindungen ausgewählt sind aus der Gruppe bestehend aus Methacrylamid-Verbindungen der allgemeinen Formel I: 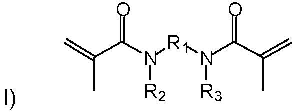 wobei R₁ eine verzweigte oder unverzweigte organische Verbindungseinheit mit 4 bis 16 Kohlenstoffatomen, bevorzugt mit 6 bis 14 Kohlenstoffatomen, besonders bevorzugt mit 8 bis 12 Kohlenstoffatomen, ist, und wobei R₂ und R₃ unabhängig voneinander Wasserstoff oder ein verzweigter oder unverzweigter organischer Rest mit 1 bis 10 Kohlenstoffatomen, bevorzugt 1 bis 6 Kohlenstoffatomen, besonders bevorzugt 1 bis 4 Kohlenstoffatomen, ist. Besonders bevorzugt ist insoweit nämlich eine erfindungsgemäße strahlungshärtbare Zusammensetzung, wobei R₁ eine aliphatische organische Verbindungseinheit ist. Besonders bevorzugt ist zusätzlich oder alternativ auch eine strahlungshärtbare Zusammensetzung, wobei R₁ eine Ringstruktur umfasst.

Bevorzugt ist mit Blick auf die zweiten (Meth)acrylamid-Verbindungen eine erfindungsgemäße strahlungshärtbare Zusammensetzung, wobei die zweiten (Meth)acrylamid-Verbindungen ausgewählt sind aus der Gruppe bestehend aus Methacrylamid-Verbindungen der allgemeinen Formel II: 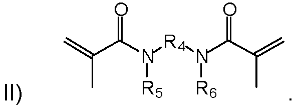 wobei R₄ eine verzweigte oder unverzweigte organische Verbindungseinheit mit 20 oder mehr, bevorzugt 30 oder mehr, besonders bevorzugt 40 oder mehr, ganz besonders bevorzugt 50 oder mehr, Atomen, ist, und wobei R₅ und R₆ unabhängig voneinander Wasserstoff oder ein verzweigter oder unverzweigter organischer Rest mit 1 bis 10 Kohlenstoffatomen, bevorzugt 1 bis 6 Kohlenstoffatomen, besonders bevorzugt 1 bis 4 Kohlenstoffatomen, ist. Besonders bevorzugt ist insoweit eine erfindungsgemäße strahlungshärtbare Zusammensetzung, wobei R₄ eine Vielzahl an Wiederholungseinheiten der gleichen Struktur umfasst. Besonders bevorzugt ist zusätzlich oder alternativ auch eine erfindungsgemäße strahlungshärtbare Zusammensetzung, wobei R₄ eine oder mehrere, bevorzugt eine Vielzahl von, Etherbindungen umfasst. Besonders bevorzugt ist wiederum zusätzlich oder alternativ zudem eine erfindungsgemäße strahlungshärtbare Zusammensetzung, wobei R₄ eine aliphatische organische Verbindungseinheit ist. Bevorzugt ist ebenfalls zusätzlich oder alternativ auch eine erfindungsgemäße strahlungshärtbare Zusammensetzung, wobei R₄ eine Vielzahl an Heteroatomen umfasst, bevorzugt Sauerstoff. Ganz besonders bevorzugt ist eine erfindungsgemäße strahlungshärtbare Zusammensetzung, wobei R₄ ein Polyglykol umfasst, bevorzugt Polypropylenglykol.

Weiterführende Informationen zur Synthese derartiger (Meth)acrylamid-Verbindungen sind beispielsweise in der EP 4378967 A1 offenbart.

Hierauf aufbauend haben die Erfinder besonders bevorzugte Verbindungen identifiziert, mit denen sich in den Experimenten der Erfinder ausgezeichnete Ergebnisse erzielen ließen. Bevorzugt ist nämlich eine erfindungsgemäße strahlungshärtbare Zusammensetzung, wobei die ersten (Meth)acrylamid-Verbindungen ausgewählt sind aus der Gruppe bestehend aus Methacrylamiden von 2,5-Bis(aminomethyl)-bicyclo-[2.2.1]-heptan, 2,6-Bis(aminomethyl)-bicyclo-[2.2.1]-heptan, 1,3-Cyclohexandimethanamin und 3-Aminomethyl-3,5,5-trimethylcyclohexylamin. Bevorzugt ist insoweit zusätzlich oder alternativ eine erfindungsgemäße strahlungshärtbare Zusammensetzung, wobei die zweiten (Meth)acrylamid-Verbindungen ausgewählt sind aus der Gruppe bestehend aus (Meth)acrylamiden von Poly(propylenglycol)-bis(2-aminopropylether).

Die erfindungsgemäßen Zusammensetzungen sind strahlungshärtbar. Der Ausdruck "strahlungshärtbar", der vorstehend zur Charakterisierung der Zusammensetzung verwendet wird, entspricht dem in der Branche üblichen Fachterminus, wobei alternativ auch gleichwertig Begriffe wie "strahlungshärtend", "strahlungsvernetzend" oder ähnliche Ausdrücke verwendet werden. Der Ausdruck ist dabei zu verstehen als die Eigenschaft der Zusammensetzung durch die Applikation von elektromagnetischer Strahlung, insbesondere im Bereich des ultravioletten bzw. sichtbaren Lichts, auszuhärten, indem durch die Strahlung mittels eines Initiators die Polymerisation von radikalisch polymerisierbaren Monomeren in der Zusammensetzung induziert wird. Die Wellenlänge der hierfür verwendeten Strahlung liegt zumeist im Bereich des sichtbaren Lichtes bzw. der angrenzenden Wellenlängenbereiche im UV-Bereich, wobei besonders häufig Wellenlängen im blauen und ultravioletten Bereich zum Einsatz kommen. In Übereinstimmung mit dem fachmännischen Verständnis wird die Strahlungshärtbarkeit durch die vorstehend definierte Kombination von (Meth)acrylamid-Verbindungen, welche über die Doppelbindungen radikalisch polymerisierbar sind, und ggf. anderen strahlungsvernetzbaren Monomeren mit Photoinitiatoren erreicht. Bevorzugt ist hinsichtlich der zur Aushärtung einsetzbaren elektromagnetischen Strahlung eine erfindungsgemäße strahlungshärtbare Zusammensetzung, wobei die Photoinitiatoren die Aushärtung der strahlungshärtbaren Zusammensetzung bei Bestrahlung mit elektromagnetischer Strahlung im Wellenlängenbereich von 200 und 500 nm, bevorzugt im Wellenlängenbereich von 350 bis 480 nm, besonders bevorzugt im Wellenlängenbereich von 380 und 470 nm, initiieren können, und/oder wobei die strahlungshärtbare Zusammensetzung bei Bestrahlung mit elektromagnetischer Strahlung im Wellenlängenbereich von 200 und 500 nm, bevorzugt im Wellenlängenbereich von 350 bis 480 nm, besonders bevorzugt im Wellenlängenbereich von 380 und 470 nm, aushärtbar ist.

Den oder die Photoinitiatoren wählt der Fachmann dabei auf Grundlage seines Fachwissens ausgehend von den eingesetzten polymerisierbaren Monomeren und im Lichte des gewünschten Wellenlängenbereichs für die Polymerisation aus, wobei er vorteilhafterweise auf tabellierte und/oder vom Hersteller bereitgestellte Informationen zurückgreifen kann. Im Rahmen der vorliegenden Erfindung umfasst der Ausdruck "Photoinitiator" auch Hilfsinitiatoren, Co-Initiatoren bzw. Beschleuniger, so lange die Gesamtheit an Verbindungen im "Photoinitiator" geeignet ist, als Photoinitiator zu wirken. Bevorzugt ist zur Einstellung der Aushärtungskinetik in vielen Fällen eine erfindungsgemäße strahlungshärtbare Zusammensetzung, wobei die strahlungshärtbare Zusammensetzung zusätzlich umfasst:
g) einen oder mehrere Coinitiatoren, bevorzugt in einem kombinierten Massenanteil im Bereich von 0,001 bis 10 %, besonders bevorzugt im Bereich von 0,01 bis 5 %, ganz besonders bevorzugt im Bereich von 0,1 bis 2 %.

Geeignete Photoinitiatoren werden beispielsweise in der EP 3020361 B1 oder der WO 95/13565 A1 offenbart, sind dem Fachmann aber auch ausgehend von seinem Fachwissen geläufig und von zahlreichen Anbietern zu diesem Zweck kommerziell erhältlich. Bevorzugt ist hinsichtlich des Gehalts an diesen Photoinitiatoren eine erfindungsgemäße strahlungshärtende Zusammensetzung, wobei der kombinierte Massenanteil der Photoinitiatoren im Bereich von 0,01 bis 5 %, bevorzugt im Bereich von 0,1 bis 2 %, besonders bevorzugt im Bereich von 0,2 bis 1%, liegt.

Neben den erfindungsgemäß einzusetzenden ersten und zweiten (Meth)acrylamid-Verbindungen können in den erfindungsgemäßen strahlungshärtbaren Zusammensetzungen auch weitere polymerisierbare Monomere eingesetzt werden, insbesondere (Meth)acrylat-Verbindungen. Hierdurch lassen sich in vorteilhafter Weise die physikalisch-chemischen Eigenschaften der strahlungshärtbaren Zusammensetzung sowie deren Handhabungseigenschaften und insbesondere auch deren Aushärtekinetik gezielt an die jeweiligen Anwendungserfordernisse des Anwendungsfalls abstimmen. Bevorzugt ist entsprechend eine erfindungsgemäße strahlungshärtbare Zusammensetzung, wobei die strahlungshärtbare Zusammensetzung zusätzlich umfasst:
d) eine oder mehrere (Meth)acrylat-Verbindungen, insbesondere (Meth)acrylatVerbindungen, welche keine Anhydrid-Gruppe und keine aziden H-X-Bindungen aufweisen, in denen X ein von Kohlenstoff verschiedenes Heteroatom ist, wobei die (Meth)acrylat-Verbindungen bevorzugt Methacrylat-Verbindungen sind.

Auch hinsichtlich der Dosierung der (Meth)acrylat-Verbindung ist es den Erfindern gelungen, besonders geeignete Bereiche zu identifizieren. Bevorzugt ist nämlich eine erfindungsgemäße strahlungshärtbare Zusammensetzung, wobei der kombinierte Massenanteil der einen oder der mehreren (Meth)acrylatVerbindungen 2 % oder mehr, bevorzugt 4 % oder mehr, besonders bevorzugt 6 % oder mehr, beträgt, bezogen auf die Gesamtmasse der strahlungshärtbaren Zusammensetzung, und/oder wobei der kombinierte Massenanteil der einen oder der mehreren (Meth)acrylat-Verbindungen 25 % oder weniger, bevorzugt 20 % oder weniger, besonders bevorzugt 15 % oder weniger, beträgt, bezogen auf die Gesamtmasse der strahlungshärtbaren Zusammensetzung. Besonders bevorzugt ist dabei eine erfindungsgemäße strahlungshärtbare Zusammensetzung, wobei der kombinierte Massenanteil der einen oder der mehreren (Meth)acrylatVerbindungen im Bereich von 2 bis 25 %, bevorzugt im Bereich von 4 bis 20 %, besonders bevorzugt im Bereich von 6 bis 15 %, liegt, bezogen auf die Gesamtmasse der strahlungshärtbaren Zusammensetzung.

Grundsätzlich bevorzugt ist eine erfindungsgemäße strahlungshärtbare Zusammensetzung, wobei die eine oder die mehreren (Meth)acrylatVerbindungen ausgewählt sind aus der Gruppe bestehend aus difunktionellen (Meth)acrylat-Verbindungen. Bevorzugt ist zusätzlich oder alternativ eine erfindungsgemäße strahlungshärtbare Zusammensetzung, wobei die eine oder die mehreren (Meth)acrylat-Verbindungen ausgewählt sind aus der Gruppe bestehend aus Urethandimethacrylat, Tricyclodecandimethanol Urethan Diacrylate, Tricyclodecandimethanol Diacrylate, Bisphenol A-Ethoxylatdimethacrylat und Bisphenol A-Glycidylmethacrylat.

Eine weitere Komponente für eine erfindungsgemäße strahlungshärtbare Zusammensetzung, mit der besonders vorteilhafte adhäsive Wirkungen erzielt werden können, insbesondere zu Dentin und Enamel, sind solche polymerisierbaren sauren Verbindungen, welche eine oder mehrere saure funktionelle Gruppen umfassen. Bevorzugt ist zusätzlich oder alternativ entsprechend eine erfindungsgemäße strahlungshärtbare Zusammensetzung, wobei die strahlungshärtbare Zusammensetzung zusätzlich umfasst:
e) eine oder mehrere polymerisierbare sauren Verbindungen, wobei die polymerisierbaren sauren Verbindungen eine Anhydrid-Gruppe oder eine azide H-X-Bindung umfassen, in der X ein von Kohlenstoff verschiedenes Heteroatom ist.

Bevorzugt ist zusätzlich oder alternativ entsprechend eine erfindungsgemäße strahlungshärtbare Zusammensetzung, wobei die eine oder die mehreren polymerisierbaren sauren Verbindungen ausgewählt sind aus der Gruppe bestehend aus (Meth)acrylat-Verbindungen, bevorzugt monofunktionellen (Meth)acrylat-Verbindungen.

Während die vorstehend definierten (Meth)acrylat-Verbindungen bevorzugt frei von sauren Gruppen sind, sind die polymerisierbaren sauren Verbindungen bewusst azide. Bevorzugt ist somit beim Vorliegen beider Verbindungsklassen zusätzlich oder alternativ entsprechend eine erfindungsgemäße strahlungshärtbare Zusammensetzung, wobei die (Meth)acrylat-Verbindungen von den polymerisierbaren sauren Verbindungen Verbindungen verschieden sind.

Nach Einschätzung der Erfinder eignen sich bestimmte Arten von sauren funktionellen Gruppen dabei ganz besonders für den Einsatz in erfindungsgemäßen aushärtbaren Zusammensetzungen, damit mit diesen besonders vorteilhafte Haftwirkungen erzielt werden können.

Bevorzugt ist zusätzlich oder alternativ entsprechend eine erfindungsgemäße strahlungshärtbare Zusammensetzung, wobei die polymerisierbaren sauren Verbindungen eine oder mehrere saure funktionelle Gruppen umfassen, wobei die sauren funktionellen Gruppen ausgewählt sind aus der Gruppe bestehend aus Carbonsäure-Gruppen, Carbonsäureanhydrid-Gruppen, Phosphat-Gruppen, Pyrophosphat-Gruppen, Thiophosphat-Gruppen, Phosphonat-Gruppen, Sulfonsäure-Gruppen und Salzen dieser Gruppen,

bevorzugt ausgewählt sind aus der Gruppe bestehend aus Carbonsäure-Gruppen, Carbonsäureanhydrid-Gruppen, Phosphat-Gruppen, Pyrophosphat-Gruppen, Thiophosphat-Gruppen, Phosphonat-Gruppen und Sulfonsäure-Gruppen, besonders bevorzugt ausgewählt sind aus der Gruppe bestehend aus Carbonsäureanhydrid-Gruppen und Phosphat-Gruppen.

Bevorzugt ist zusätzlich oder alternativ entsprechend eine erfindungsgemäße strahlungshärtbare Zusammensetzung, wobei die eine oder die mehreren polymerisierbaren sauren Verbindungen ausgewählt sind aus der Gruppe bestehend aus 10-(Phosphonooxy)decyl-(meth)acrylat, Glycerindimethacrylatphosphat, 2-Phosphonooxy)propane-1,3-diylbismethacrylate, Bis(2-methacryloxyethyl)phosphat, 4-Methacryloyloxyethyl trimellitic acid anhydrid, 4-(2-Ethoxycarbonyl-allyloxy)-phthalsäure, 11-Methacryloyloxy-1,1-undecandicarbonsäure und methacrylierter Polycarbonsäure.

Auch hinsichtlich der Dosierung dieser vom Fachmann auch als "saure Monomere" bezeichneten Verbindungen konnten die Erfinder bevorzugte Bereiche identifizieren. Bevorzugt ist nämlich eine erfindungsgemäße strahlungshärtbare Zusammensetzung, wobei der kombinierte Massenanteil der einen oder der mehreren polymerisierbaren sauren Verbindungen 5 % oder mehr, bevorzugt 10 % oder mehr, besonders bevorzugt 15 % oder mehr, beträgt, bezogen auf die Gesamtmasse der strahlungshärtbaren Zusammensetzung, und/oder wobei der kombinierte Massenanteil der einen oder der mehreren polymerisierbaren sauren Verbindungen 35 % oder weniger, bevorzugt 30 % oder weniger, besonders bevorzugt 25 % oder weniger, beträgt, bezogen auf die Gesamtmasse der strahlungshärtbaren Zusammensetzung. Besonders bevorzugt ist entsprechend eine erfindungsgemäße strahlungshärtbare Zusammensetzung, wobei der kombinierte Massenanteil der einen oder der mehreren polymerisierbaren sauren Verbindungen im Bereich von 5 bis 35 %, bevorzugt im Bereich von 10 bis 30 %, besonders bevorzugt im Bereich von 15 bis 25 %, liegt, bezogen auf die Gesamtmasse der strahlungshärtbaren Zusammensetzung.

In vielen anderen Bereichen der Dentalchemie werden strahlungshärtbare Zusammensetzungen gezielt mit Füllstoffen versehen, um die mechanischen Eigenschaften zu optimieren. Ein entsprechendes Vorgehen ist dabei allerdings vor allem für solche Materialien angezeigt, die den Hauptteil einer Kunststofffüllung bilden und entsprechend über korrespondierende mechanische Eigenschaften verfügen sollen. Für die im Fokus der Erfindung stehenden dentalen Adhäsive liegt der Schwerpunkt hingegen anders. Zwar ist es denkbar, auch in den dentalen Adhäsiven Füllstoffe einzusetzen, jedoch erachten es die Erfinder für die weit überwiegende Zahl der Fälle als bevorzugt, den Gehalt an Füllstoffen eher gering zu halten, insbesondere auch um das Risiko für eine ungewollte Sedimentation der Füllstoffe bei der Lagerung zu verringern. Bevorzugt ist demgemäß eine erfindungsgemäße strahlungshärtbare Zusammensetzung, wobei die strahlungshärtbare Zusammensetzung bezogen auf die Masse der strahlungshärtbaren Zusammensetzung weniger als 10 %, bevorzugt weniger als 5 %, besonders bevorzugt weniger als 2 %, ganz besonders bevorzugt weniger als 1 %, an Füllstoffen umfasst.

Mit Blick auf den späteren Einsatz und die gewünschten Anwendungseigenschaften ist es für die weit überwiegende Zahl der Fälle bevorzugt, die strahlungshärtbare Zusammensetzung mit einem Lösungsmittel zu versetzen, wobei insbesondere Wasser und Alkohole hierfür besonders geeignet sind. Bevorzugt ist entsprechend eine erfindungsgemäße strahlungshärtbare Zusammensetzung, wobei die strahlungshärtbare Zusammensetzung zusätzlich umfasst:
f) ein oder mehrere Lösungsmittel mit einem Siedepunkt bei 100 kPa von 150 °C oder weniger, bevorzugt 130 °C oder weniger, besonders bevorzugt 110 °C oder weniger, bevorzugt in einem kombinierten Massenanteil im Bereich von 30 bis 70 %, besonders bevorzugt im Bereich von 35 bis 65 %, ganz besonders bevorzugt im Bereich von 40 bis 60 %.

Es handelt sich dabei angesichts der Siedepunkte in Übereinstimmung mit dem fachmännischen Verständnis entsprechend um eine erfindungsgemäße strahlungshärtbare Zusammensetzung, wobei die Lösungsmittel bei 25 °C flüssig sind und/oder einen Schmelzpunkt von 10 °C oder weniger, aufweisen.

Besonders bevorzugt ist hinsichtlich der Auswahl der Lösungsmittel eine erfindungsgemäße strahlungshärtbare Zusammensetzung, wobei das eine oder die mehreren Lösungsmittel ausgewählt sind aus der Gruppe bestehend aus Wasser und Alkoholen, bevorzugt Wasser und Ethanol.

Beim Einsatz von Lösungsmitteln wird die strahlungshärtbare Zusammensetzung als Ganzes in den meisten Fällen eine Flüssigkeit sein, was für den angestrebten Anwendungszweck als dentales Adhäsiv bevorzugt ist. Bevorzugt ist entsprechend eine erfindungsgemäße strahlungshärtbare Zusammensetzung, wobei die strahlungshärtbare Zusammensetzung bei einem Druck von 100 kPa bei 25 °C eine dynamische Viskosität gemessen gemäß DIN 53019-1 aus 2008 von 9000 mPa*s oder weniger, bevorzugt 6000 mPa*s oder weniger, besonders bevorzugt 3000 mPa*s oder weniger, aufweist.

Für einige Anwendungsfelder kann es wünschenswert sein, den Gehalt an Acrylamid-Verbindungen möglichst gering zu halten, beispielsweise weil dies den Anforderungen von Kunden entspricht, die entsprechende Anforderungen beispielsweise unter gesundheitlichen und/oder marketingtechnischen Gesichtspunkten stellen. Hierbei kann es als großer Vorteil der vorliegenden Erfindung gesehen werden, dass diese ohnehin besonders vorteilhafte Ergebnisse beim Einsatz von Methacrylamid-Verbindungen zeigt, sodass in vorteilhafter Weise auf den Einsatz von Acrylamid-Verbindungen bei Bedarf auch weitgehend verzichtet werden kann. Bevorzugt ist für manche Ausgestaltungen entsprechend eine erfindungsgemäße strahlungshärtbare Zusammensetzung, wobei die strahlungshärtbare Zusammensetzung bezogen auf die Masse der strahlungshärtbare Zusammensetzung weniger als 5 %, bevorzugt weniger als 2 %, besonders bevorzugt weniger als 1 %, ganz besonders bevorzugt weniger als 0,5 %, an Acrylamid-Verbindungen umfasst.

Die Erfindung betrifft auch eine dentale Verklebung, hergestellt oder herstellbar durch strahlungsinduzierte Polymerisation einer erfindungsgemäßen strahlungshärtbaren Zusammensetzung.

Besonders relevant ist dabei eine erfindungsgemäße dentale Verklebung, wobei die dentale Verklebung stoffschlüssig mit Dentin und/oder Enamel verbunden ist.

Besonders relevant ist zusätzlich oder alternativ hierzu eine erfindungsgemäße dentale Verklebung, wobei die dentale Verklebung stoffschlüssig mit einem polymerisierten Dentalmaterial verbunden ist.

Ganz besonders bevorzugt ist entsprechend eine erfindungsgemäße dentale Verklebung, wobei die dentale Verklebung Bestandteil einer dentalen Restauration ist.

Offenbart wird zudem die Verwendung eines (Meth)acrylamid-Gemischs bei der Herstellung von strahlungshärtbaren dentalen Adhäsiven zur Verringerung der Abnahme der Scherhaftung des dentalen Adhäsivs an Dentin und/oder Enamel, bevorzugt Dentin, mit der Zeit, wobei das Gemisch bezogen auf die Masse des herzustellenden dentalen Adhäsivs umfasst:
A) eine oder mehrere erste (Meth)acrylamid-Verbindungen in einem kombinierten Massenanteil von 1 % oder mehr,
   wobei die ersten (Meth)acrylamid-Verbindungen bei einem Druck von 100 kPa einen Schmelzpunkt von 30 °C oder mehr aufweisen, und
B) eine oder mehrere zweite (Meth)acrylamid-Verbindungen in einem kombinierten Massenanteil von 0,1 % oder mehr,
   wobei die zweiten (Meth)acrylamid-Verbindungen bei einem Druck von 100 kPa einen Schmelzpunkt von 20 °C oder weniger aufweisen.

Nachfolgend werden die Erfindung und bevorzugte Ausführungsformen der Erfindung unter Bezugnahme auf Experimente näher erläutert und beschrieben.

### A. Herstellung der strahlungshärtbaren Zusammensetzungen:

Die Herstellung der strahlungshärtbaren Zusammensetzungen erfolgte in branchenüblicher Weise durch Vermischen der Komponenten. Hierfür wurden die Bestandteile eingewogen und mit Hilfe eines Magnetrührers für 30 min bei 40 °C und dann über Nacht bei 23 °C gerührt, bis ein homogenes Gemisch erhalten wurde. Nach Zugabe des Initiators wurde darauf geachtet, dass die Zusammensetzung keiner unbeabsichtigten Belichtung ausgesetzt wird.

Für die Herstellung der untersuchten Zusammensetzungen kamen die in Tabelle 1 aufgeführten (Meth)acrylamid-Verbindungen sowie die in Tabelle 2 aufgeführten weiteren Bestandteile zum Einsatz.

**Tabelle 1 - Eingesetzte (Meth)acrylamid-Verbindungen**

| Bezeichnung | Strukturformel | Aggregatzustand bei 25 °C |
|---|---|---|
| MAM1 | 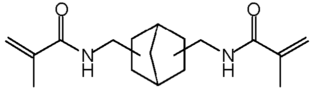 | fest (Smp. > 30 °C) |
| MAM2 | 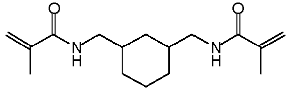 | fest (Smp. > 30 °C) |
| MAM3 | 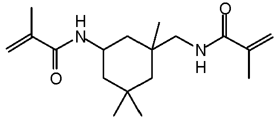 | fest (Smp. > 30 °C) |
| MAM4 | 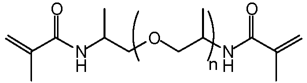 | flüssig (Smp. < 20 °C) |
| MAM5 | 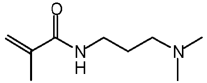 | flüssig (Smp. < 20 °C) |
| AM1 | 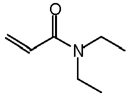 | flüssig (Smp. < 20 °C) |

**Tabelle 2 - Eingesetzte weitere Bestandteile**

| Bezeichnung | Verbindung |
|---|---|
| UDMA | Urethandimethacrylat |
| Adhäsivmonomer | Gemisch: 4-Methacryloxyethyltrimellitateanhydrid (4-META) & 10-Methacryloyloxydecyldihydrogenphosphat (10-MDP) |
| Lösungsmittel | Gemisch: Wasser & Ethanol |
| Photoinitiator | Campherchinon |
| Co-Initiator | Gemisch: Handelsprodukt Speedcure BEDB & Handelsprodukt Omnicat 440 |
| Verdicker | Ethylcellulose |
| Stabilisator | 2,5-Di-tert-butylhydroquinone (DTBHQ), Butylhydroxytoluol (BHT) |

Die hergestellten strahlungshärtbaren Zusammensetzungen sind in den Tabellen 3 und 4 zusammengestellt.

**Tabelle 3 - Hergestellte Zusammensetzungen - Teil 1 (alle Angaben in Massenanteilen / %)**

| Bezeichnung | V1 | V2 | V3 | V4 | V5 | V6 |
|---|---|---|---|---|---|---|
| MAM1 | 13,0 | | | | | |
| MAM2 | | 13,0 | | | | |
| MAM3 | | | 13,0 | | | |
| MAM4 | | | | 13,0 | | |
| MAM5 | | | | | 13,0 | |
| AM1 | | | | | | 13,0 |
| UDMA | 11,0 | 11,0 | 11,0 | 11,0 | 11,0 | 11,0 |
| Adhäsivmonomer | 18,6 | 18,6 | 18,6 | 18,6 | 18,6 | 18,6 |
| Lösungsmittel | 55,0 | 55,0 | 55,0 | 55,0 | 55,0 | 55,0 |
| Photoinitiator | 0,6 | 0,6 | 0,6 | 0,6 | 0,6 | 0,6 |
| Co-Initiator | 0,9 | 0,9 | 0,9 | 0,9 | 0,9 | 0,9 |
| Verdicker | 0,75 | 0,75 | 0,75 | 0,75 | 0,75 | 0,75 |
| Stabilisator | 0,15 | 0,15 | 0,15 | 0,15 | 0,15 | 0,15 |

**Tabelle 4 - Hergestellte Zusammensetzungen - Teil 2 (alle Angaben in Massenanteilen / %)**

| Bezeichnung | E1 | E2 | E3 | E4 | E5 |
|---|---|---|---|---|---|
| MAM1 | 11,0 | | | 11,0 | 11,0 |
| MAM2 | | 11,0 | | | |
| MAM3 | | | 11,0 | | |
| MAM4 | 2,0 | 2,0 | 2,0 | | |
| MAM5 | | | | 2,0 | |
| AM1 | | | | | 2,0 |
| UDMA | 11,0 | 11,0 | 11,0 | 11,0 | 11,0 |
| Adhäsivmonomer | 18,6 | 18,6 | 18,6 | 18,6 | 18,6 |
| Lösungsmittel | 55,0 | 55,0 | 55,0 | 55,0 | 55,0 |
| Photoinitiator | 0,6 | 0,6 | 0,6 | 0,6 | 0,6 |
| Co-Initiator | 0,9 | 0,9 | 0,9 | 0,9 | 0,9 |
| Verdicker | 0,75 | 0,75 | 0,75 | 0,75 | 0,75 |
| Stabilisator | 0,15 | 0,15 | 0,15 | 0,15 | 0,15 |

Von den hergestellten strahlungshärtbaren Zusammensetzungen sind die strahlungshärtbaren Zusammensetzungen V1, V2, V3, V4, V5 und V6 nicht erfindungsgemäß. Die strahlungshärtbaren Zusammensetzungen E1, E2, E3, E4 und E5 sind erfindungsgemäß.

### B. Herstellung der Prüfkörper:

Aus den in den Tabellen 3 und 4 aufgeführten strahlungshärtbaren Zusammensetzungen wurden Prüfkörper hergestellt, an denen anschließend die Scherhaftung bestimmt wurde.

Die Prüfkörper wurden entsprechend der Norm ISO 29022:2013(E) hergestellt. Als Substrat wurden konservierte, kariesfreie Rinderzähne verwendet, die im Kühlschrank in Wasser gelagert wurden. Nach dem Einbetten in ein Fixierungsmaterial (Handelsname: Technovit) werden die Zähne nass geschliffen um das Dentin freizulegen. Hierfür wurde Schleifpapier mit grober (P120) und feiner (P400) Körnung und eine hydraulische Schleifmaschine (Fa. Buehler, Handelsname: Phönix 4000) verwendet. Die präparierten Zähne wurden anschließend kurzzeitig in Wasser gelagert und innerhalb einer Stunde für die weitere Vorbereitung verwendet. Vor Auftragen der strahlungshärtbaren Zusammensetzungen wurden die präparierten Zähne mit frischem Wasser abgespült und das oberflächliche Wasser entfernt. Anschließend wurden die strahlungshärtbaren Zusammensetzungen mit einem Applikatortip auf die zu beklebende Stelle Dentin aufgetragen und 20 s einmassiert.

Nach dem Einwirken und der damit verbundenen Ätzung wurde das verbliebene Lösungsmittelgemisch mit Druckluft entfernt und der resultierende Adhäsivfilm mithilfe einer Dentallampe (Fa. Kulzer, Handelsname: Translux 2Wave) für 10 s belichtet. Nach der initialen Belichtung wurde ein dentales Kompositmaterial (Fa. Kulzer, Handelsname: Venus Diamond) mithilfe einer Vorrichtung in Form eines Zylinders auf dem Adhäsivfilm platziert. Das Kompositmaterial wurde für 20 s belichtet, wobei die selbe Dentallampe verwendet wurde. Nach Aushärtung des Komposits wird die Vorrichtung entfernt und der fertige Prüfkörper in Wasser zwischengelagert.

Die Lagerung erfolgte für die nachfolgenden Experimente mit einer von zwei Lagerungsarten:

| | |
|---|---|
| Lagerung I | Wasserlagerung für 24 Stunden bei 37 °C; oder |
| Lagerung II | Wasserlagerung für 24 Stunden bei 37 °C, mit anschließender Thermowechsellast (Fa. SD Mechatronik, Handelsname: Thermocycler) in einem Temperaturbereich zwischen 5 °C und 55 °C mit 5.000 Zyklen. |

### C. Bestimmung der Scherhaftung

Die Scherfestigkeit der Kompositmaterialien am Dentin wurde an den gelagerten Prüfkörpern entsprechend der Norm ISO 29022:2013(E) an einer Universalprüfmaschine (Fa. Zwick Roell, Handelsname: Z010) gemessen.

Die Scherfestigkeit wurde jeweils als arithmetischer Mittelwert aus 8 Messungen erhalten.

Die Ergebnisse sind in der nachfolgenden Tabelle 5 zusammengestellt.

**Tabelle 5 - Bestimmte Scherfestigkeiten**

| Probe | Scherfestigkeit / MPa (Lagerung I) | Scherfestigkeit / MPa (Lagerung II) |
|---|---|---|
| V1 | 23,2 | |
| V2 | 13,1 | |
| V3 | 18,8 | |
| V4 | 28,2 | 23,7 |
| V5 | 14,1 | |
| V6 | 27,4 | |
| E1 | 27,1 | 27,6 |
| E2 | 27,7 | |
| E3 | 29,2 | |
| E4 | 27,0 | 30,1 |
| E5 | 31,0 | |

### D. Bewertung:

Die in Tabelle 5 zusammengestellten Versuchsergebnisse zeigen, dass für viele der nicht-erfindungsgemäßen, strahlungshärtbaren Zusammensetzungen nur unzureichende initiale Scherfestigkeiten erreicht werden können, insbesondere für die festen (Meth)acrylamid-Verbindungen.

Zudem zeigt die Messung nach Thermowechsellast für die Probe V4, dass sich die Scherfestigkeit für die flüssige (Meth)acrylamid-Verbindung MAM4 unter Belastung verschlechtert.

Der Vergleich mit den Proben E1 bis E5 zeigt eindrucksvoll, dass durch die Mischung der (Meth)acrylamid-Verbindungen signifikant erhöhte initiale Scherfestigkeiten erreicht werden können, insbesondere auch für die festen (Meth)acrylamid-Verbindungen MAM2 und MAM3, für welche sich besonders niedrige initiale Scherfestigkeiten ergeben haben.

Besonders überraschend ist dabei, dass sich kein lediglich linearer Beitrag der einzelnen (Meth)acrylamid-Verbindungen ergibt, sondern die für die Mischung erhaltenen Scherfestigkeiten signifikant über denen liegen, die aus der linearen Extrapolation zu erwarten wären. Entsprechend zeigt sich ein synergistischer Effekt, welcher es erlaubt, bereits bei geringen Mengen an flüssigen (Meth)acrylamid-Verbindungen einen vorteilhaften Effekt zu erzielen.

Hierbei ist bemerkenswert, dass sich der vorteilhafte Effekt der Mischung sogar mit den flüssig (Meth)acrylamid-Verbindungen MAM5 zeigt, die bei alleinigem Einsatz selbst eine niedrige initiale Scherfestigkeit zeigen. Dass die Kombination von MAM1 (23,2 MPa) und MAM5 (14,1 MPa) in der Mischung eine Scherfestigkeit von 27,0 MPa ergibt, ist völlig unerwartet.

Zudem zeigen die Werte, dass sich für die Proben E1 und E4 auch nach Lagerung bei Thermowechsellast ausgezeichnete Ergebnisse erzielen lassen und dass die Verschlechterung der Scherfestigkeit, die unter diesen Bedingungen für V4 beobachtet wird, vermieden werden kann.

## Patentansprüche

1. Strahlungshärtbare Zusammensetzung für den Einsatz als dentales Adhäsiv, umfassend bezogen auf die Gesamtmasse der strahlungshärtbaren Zusammensetzung:
a) eine oder mehrere erste (Meth)acrylamid-Verbindungen in einem kombinierten Massenanteil von 1 % oder mehr,
wobei die ersten (Meth)acrylamid-Verbindungen bei einem Druck von 100 kPa einen Schmelzpunkt von 30 °C oder mehr aufweisen,
b) eine oder mehrere zweite (Meth)acrylamid-Verbindungen in einem kombinierten Massenanteil von 0,1 % oder mehr,
wobei die zweiten (Meth)acrylamid-Verbindungen bei einem Druck von 100 kPa einen Schmelzpunkt von 20 °C oder weniger aufweisen, und
c) einen oder mehrere Photoinitiatoren in einem kombinierten Massenanteil im Bereich von 0,001 bis 10 %.

2. Strahlungshärtbare Zusammensetzung nach Anspruch 1, wobei der kombinierte Massenanteil der einen oder der mehreren ersten (Meth)acrylamid-Verbindungen im Bereich von 2 bis 30 %, bevorzugt im Bereich von 4 bis 25 %, bevorzugt im Bereich von 6 bis 25 %, liegt, ganz besonders bevorzugt im Bereich von 10 bis 15 %, bezogen auf die Gesamtmasse der strahlungshärtbaren Zusammensetzung,
und/oder
wobei der kombinierte Massenanteil der einen oder der mehreren zweiten (Meth)acrylamid-Verbindungen im Bereich von 0,2 bis 10 %, bevorzugt im Bereich von 0,4 bis 8 %, bevorzugt im Bereich von 0,6 bis 6 %, liegt, ganz besonders bevorzugt im Bereich von 0,8 bis 4 %, bezogen auf die Gesamtmasse der strahlungshärtbaren Zusammensetzung.

3. Strahlungshärtbare Zusammensetzung nach einem der Ansprüche 1 oder 2, wobei der Quotient aus dem kombinierten Massenanteil an ersten (Meth)acrylamid-Verbindungen geteilt durch den kombinierten Massenanteil an zweiten (Meth)acrylamid-Verbindungen 1 oder mehr, bevorzugt 2 oder mehr, besonders bevorzugt 4 oder mehr, beträgt.

4. Strahlungshärtbare Zusammensetzung nach einem der Ansprüche 1 bis 3, wobei der Quotient aus dem kombinierten Massenanteil an ersten (Meth)acrylamid-Verbindungen geteilt durch den kombinierten Massenanteil an zweiten (Meth)acrylamid-Verbindungen 20 oder weniger, bevorzugt 15 oder weniger, besonders bevorzugt 10 oder weniger, beträgt.

5. Strahlungshärtbare Zusammensetzung nach einem der Ansprüche 1 bis 4, wobei die ersten (Meth)acrylamid-Verbindungen bei einem Druck von 100 kPa einen Schmelzpunkt von 40 °C oder mehr, bevorzugt 50 °C oder mehr, besonders bevorzugt 60 °C oder mehr, aufweisen,
und/oder
wobei die zweiten (Meth)acrylamid-Verbindungen bei einem Druck von 100 kPa einen Schmelzpunkt von 10 °C oder weniger, bevorzugt 0 °C oder weniger, besonders bevorzugt -10 °C oder weniger, aufweisen.

6. Strahlungshärtbare Zusammensetzung nach einem der Ansprüche 1 bis 5, wobei die ersten (Meth)acrylamid-Verbindungen ausgewählt sind aus der Gruppe bestehend aus Methacrylamiden.

7. Strahlungshärtbare Zusammensetzung nach einem der Ansprüche 1 bis 6, wobei die zweiten (Meth)acrylamid-Verbindungen ausgewählt sind aus der Gruppe bestehend aus Methacrylamiden.

8. Strahlungshärtbare Zusammensetzung nach einem der Ansprüche 1 bis 7, wobei die ersten (Meth)acrylamid-Verbindungen ausgewählt sind aus der Gruppe bestehend aus Methacrylamid-Verbindungen der allgemeinen Formel I: 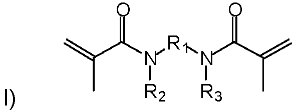
wobei R₁ eine verzweigte oder unverzweigte organische Verbindungseinheit mit 4 bis 16 Kohlenstoffatomen, bevorzugt mit 6 bis 14 Kohlenstoffatomen, besonders bevorzugt mit 8 bis 12 Kohlenstoffatomen, ist,
wobei R₂ und R₃ unabhängig voneinander Wasserstoff oder ein verzweigter oder unverzweigter organischer Rest mit 1 bis 10 Kohlenstoffatomen, bevorzugt 1 bis 6 Kohlenstoffatomen, besonders bevorzugt 1 bis 4 Kohlenstoffatomen, ist.

9. Strahlungshärtbare Zusammensetzung nach einem der Ansprüche 1 bis 8, wobei die zweiten (Meth)acrylamid-Verbindungen ausgewählt sind aus der Gruppe bestehend aus Methacrylamid-Verbindungen der allgemeinen Formel II: 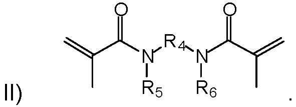
wobei R₄ eine verzweigte oder unverzweigte organische Verbindungseinheit mit 20 oder mehr, bevorzugt 30 oder mehr, besonders bevorzugt 40 oder mehr, ganz besonders bevorzugt 50 oder mehr, Atomen, ist
wobei R₅ und R₆ unabhängig voneinander Wasserstoff oder ein verzweigter oder unverzweigter organischer Rest mit 1 bis 10 Kohlenstoffatomen, bevorzugt 1 bis 6 Kohlenstoffatomen, besonders bevorzugt 1 bis 4 Kohlenstoffatomen, ist.

10. Strahlungshärtbare Zusammensetzung nach einem der Ansprüche 1 bis 9, wobei die ersten (Meth)acrylamid-Verbindungen ausgewählt sind aus der Gruppe bestehend aus Methacrylamiden von 2,5-Bis(aminomethyl)-bicyclo-[2.2.1]-heptan, 2,6-Bis(aminomethyl)-bicyclo-[2.2.1]-heptan, 1,3-Cyclohexandimethanamin und 3-Aminomethyl-3,5,5- trimethylcyclohexylamin.

11. Strahlungshärtbare Zusammensetzung nach einem der Ansprüche 1 bis 10, wobei die zweiten (Meth)acrylamid-Verbindungen ausgewählt sind aus der Gruppe bestehend aus (Meth)acrylamiden von Poly(propylenglycol)-bis(2-aminopropylether).

12. Strahlungshärtbare Zusammensetzung nach einem der Ansprüche 1 bis 11, wobei die strahlungshärtbare Zusammensetzung zusätzlich umfasst:
d) ein oder mehrere (Meth)acrylat-Verbindungen, insbesondere (Meth)acrylatVerbindungen, welche keine Anhydrid-Gruppe und keine aziden H-X-Bindungen aufweisen, in denen X ein von Kohlenstoff verschiedenes Heteroatom ist, wobei die (Meth)acrylat-Verbindungen bevorzugt Methacrylat-Verbindungen sind.

13. Strahlungshärtbare Zusammensetzung nach einem der Ansprüche 1 bis 12, wobei die strahlungshärtbare Zusammensetzung zusätzlich umfasst:
e) ein oder mehrere polymerisierbare saure Verbindungen, wobei die polymerisierbaren sauren Verbindungen eine Anhydrid-Gruppe oder eine azide H-X-Bindung umfassen, in der X ein von Kohlenstoff verschiedenes Heteroatom ist.

14. Strahlungshärtbare Zusammensetzung nach einem der Ansprüche 1 bis 13, wobei die strahlungshärtbare Zusammensetzung zusätzlich umfasst:
f) ein oder mehrere Lösungsmittel mit einem Siedepunkt bei 100 kPa von 150 °C oder weniger, bevorzugt 130 °C oder weniger, besonders bevorzugt 110 °C oder weniger, bevorzugt in einem kombinierten Massenanteil im Bereich von 30 bis 70 %, besonders bevorzugt im Bereich von 35 bis 65 %, ganz besonders bevorzugt im Bereich von 40 bis 60 %.

15. Dentale Verklebung, hergestellt oder herstellbar durch strahlungsinduzierte Polymerisation einer strahlungshärtbaren Zusammensetzung nach einem der Ansprüche 1 bis 14.
